# EUROPÄISCHE PATENTSCHRIFT

(11) **EP 1 658 256 B1**
(45) Veröffentlichungstag und Bekanntmachung des Hinweises auf die Patenterteilung: **30.05.2007**
(21) Anmeldenummer: 04763087.6
(22) Anmeldetag: 03.07.2004
(51) Int. Cl.: C07C 29/42, C07C 33/042

(54) **VERFAHREN ZUR HERSTELLUNG VON PROPARGYLALKOHOL**
METHOD FOR THE PRODUCTION OF PROPARGYL ALCOHOL
PROCEDE DE PRODUCTION D'ALCOOL PROPARGYLIQUE

(30) Priorität: 24.07.2003 DE 10333598
(43) Veröffentlichungstag der Anmeldung: 24.05.2006
(73) Patentinhaber: BASF Aktiengesellschaft, 67056 Ludwigshafen (DE)
(72) Erfinder: VICARI, Maximilian, 67117 Limburgerhof (DE); RUDLOFF, Martin, 67161 Gönnheim (DE); KRAMER, Andreas, 67159 Friedelsheim (DE); DREWS, Ronald, 69488 Birkenau (DE)
(86) Internationale Anmeldenummer: PCT/EP2004/007269
(87) Internationale Veröffentlichungsnummer: WO 2005/019144

(56) Entgegenhaltungen:
- DE-B- 1 284 964
- US-A- 3 087 970

## Beschreibung

Die Erfindung betrifft ein Verfahren zur Herstellung von Propargylalkohol durch Umsetzung einer Acetylen enthaltenden wässrigen Formaldehydlösung in Gegenwart von Kupferacetylid enthaltenden Katalysatoren bei einem Betriebsdruck von 1 bis 15 bar und 70 bis 120°C ohne Ausbildung einer zusammenhängenden Gasphase, bei dem die wässrige Formaldehydlösung ein organisches Lösungsmittel für Acetylen enthält und der Katalysator im Fließbett angeordnet ist.

Die Ethinylierung zur Herstellung von Alkinolen ist ein seit langem bekannter Prozess. Sein Hauptnachteil ist die Gefährlichkeit des gasförmigen Acetylens, der bekanntlich dazu zwingt, kostspielige Sicherheitsmaßnahmen zu ergreifen, zum Beispiel die Apparatur für das mehr als Zehnfache des Betriebsdrucks auszulegen.

Um das Verfahren möglichst sicher und wirtschaftlich zu betreiben, wurde daher in DE-A 24 21 407 vorgeschlagen, dass nur in den Anlagenteilen zur Acetylenverdichtung eine freie zusammenhängende Gasphase auftritt. Die Synthesereaktoren werden jedoch nur mit in Formaldehydlösung gelöstem Acetylen betrieben. Gemäß DE-A 24 21 407 entsteht unter diesen Bedingungen hauptsächlich 1,4-Butindiol.

DE-A 1 284 964 offenbart ein Verfahren zur Herstellung von Propargylalkohol an einem Festbettkatalysator, bei dem gegebenenfalls ein Lösungsmittel zugesetzt werden kann. Generell müssen bei der Durchführung in Festbettreaktoren sehr große Reaktorvolumina und/oder sehr lange Reaktionszeiten in Kauf genommen werden, da die Produktivität von Festbettkatalysatoren relativ gering ist. Zudem ist der Austausch verbrauchter Katalysatoren mit erheblichem Aufwand verbunden und erfordert lange Stillstandzeiten.

Um die Aktivität der Katalysatoren bestmöglich zu nutzen, werden auch kleine Katalysatorpartikel bis hin zu Pulvern eingesetzt, die im Reaktionsmedium suspendiert werden. Die Abtrennung des Katalysators vom Reaktionsmedium erfordert hier jedoch einen großen technischen Aufwand.

So ist aus US-A 4 117 248 bekannt, dass die Umsetzung von Formaldehyd und Acetylen in Suspension vorteilhaft ist. Nachteilig an dieser Fahrweise ist jedoch, dass eine zusammenhängende Acetylen-Gasphase auftritt und nur 0,7 Gew.-% Propinol entstehen, die nicht wirtschaftlich aus dem Reaktionsgemisch abgetrennt werden können.

Auch US 3,078,970 offenbart ein von einer zusammenhängenden Acetylen-Gasphase freies Verfahren zur Herstellung von Propargylalkohol unter Verwendung von N-Alkylpyrrolidon in Suspension. Nachteilig ist wiederum insbesondere der in Suspension befindliche feinteilige Katalysator, der eine aufwendige und störanfällige Abtrennung, zum Beispiel durch Filtration oder Zentrifugieren, erforderlich macht.

Die Wirtschaftlichkeit eines heterogen-katalysierten Ethinylierungsverfahrens hängt jedoch entscheidend von der Produktivität des Katalysators, seiner leichten Abtrennbarkeit aus dem Reaktionsmedium und einer hohen Anlagenverfügbarkeit ab.

Der vorliegenden Erfindung lag daher die Aufgabe zugrunde, ein Verfahren zur Herstellung von Propargylalkohol bereitzustellen, welches die einfache und sichere Herstellung durch Ethinylierung ermöglicht und die Nachteile der Verfahren nach dem Stand der Technik vermeidet. Dabei ist es besonders gewünscht, dass eine hohe Raum-Zeit-Ausbeute bei einfacher Katalysatorabtrennung ermöglicht wird.

Die Aufgabe wird erfindungsgemäß gelöst durch ein Verfahren zur Herstellung von Propargylalkohol durch Umsetzung einer Acetylen enthaltenden wässrigen Formaldehyd-Lösung an einem Kupferacetylid aufweisenden Katalysator bei einem Betriebsdruck von 1 bis 15 bar und 70 bis 120°C ohne Ausbildung einer zusammenhängenden Gasphase, dadurch gekennzeichnet, dass die wässrige Formaldehydlösung ein organisches Lösungsmittel für Acetylen enthält und der Katalysator im Fließbett angeordnet ist.

Die Katalysatorpartikel werden dabei erfindungsgemäß vorzugsweise durch das Durchströmen mit Reaktionsmedium auf eine bestimmte Weise fluidisiert.

Bei der bevorzugten Ausführungsform der Erfindung wird die Fluidisierung derart durchgeführt, dass eine Ausbildung eines expandierten Flüssigkeits-Feststoff Fließbetts erzielt wird. Diese geht einher mit einer deutlichen Ausdehnung des Katalysatorbetts, einer entsprechenden Zunahme des Lückenvolumens zwischen den Katalysatorpartikeln und einer deutlichen Rückvermischung der Katalysatorpartikel.

In einer anderen Ausführungsform wird die Fluidisierung derart durchgeführt, dass die Ausdehnung des Katalysatorbetts und die Zunahme des Lückenvolumens zwischen den Katalysatorpartikein gering bleibt. Damit erhalten die Katalysatorpartikel eine gewisse Beweglichkeit. Es soll jedoch dabei keine makroskopische Durchmischung des Katalysatorbetts eintreten. Dies wird durch eine Fluidisierung des Katalysatorbetts am Lockerungspunkt erreicht. Bei einem Fliessbett jenseits des Lockerungspunktes - etwa bei einem expandierten Fliessbett - tritt eine makroskopische Vermischung ein.

In beiden Ausführungsformen wird die Fluidisierung des Katalysators so durchgeführt, dass keine größeren Mengen an Katalysator aus dem Ethinylierungsreaktor ausgetragen werden. Erreicht wird dieses Verhalten durch eine geeignet gewählte Durchströmung des Katalysatorbetts. Die optimale Durchströmung, ausgedrückt beispielsweise durch die Leerrohrgeschwindigkeit, muss an die gewünschte Ausführungsform der Erfindung (expandiertes Fliessbett oder Fliessbett am Lockerungspunkt), die Viskosität und Dichte des Reaktionsmediums sowie die Eigenschaften der Katalysatorpartikel, insbesondere deren Größe, Form, Dichte und Porosität, angepasst werden.

Eine zu geringe Leerrohrgeschwindigkeit führt zu einem Verlust der Fluidisierung. Mit dem Erreichen der für die Minimalfluidisierung notwendigen Leerrohrgeschwindigkeit wird ein dauerndes Lösen und Bilden von Feststoffkontakten erreicht, das für ein Fliessbett am Lockerungspunkt charakteristisch ist. Eine Erhöhung der Leerrohrgeschwindigkeit führt zur einer Vergrößerung des Abstandes zwischen den Partikeln sowie zu einer höheren Beweglichkeit der Partikel und damit zu einer makroskopischen Durchmischung des Katalysatorbetts (expandiertes Fliessbett). Zu hohe Leerrohrgeschwindigkeiten führen schließlich zu einem massiven Austrag von Katalysatorpartikefn mit dem Reaktionsmedium aus dem Reaktor.

Die optimalen Parameter für das erfindungsgemäße Verfahren am Lockerungspunkt können theoretisch oder experimentell ermittelt werden. Als experimentelles Verfahren zum Auffinden des angestrebten Lockerungspunktes eignet sich die Analyse des Druckverlustes über die Katalysatorschüttung in Abhängigkeit der Leerrohrgeschwindigkeit. Bei zu geringen Leerrohrgeschwindigkeiten steigt der Druckverlust mit der Strömungsgeschwindigkeit - entsprechend den Verhältnissen bei einem Festbett - kontinuierlich an; das Bett befindet sich noch nicht im fluidisierten Zustand. Ab dem gesuchten Lockerungspunkt (Minimalfluidisations-Geschwindigkeit) ist der Anstieg des Druckverlusts hingegen deutlich geringer bzw. bleibt konstant.

Ein Maß für das Vorliegen eines Fliessbetts am Lockerungspunkt bzw. expandierten Fliessbettes ist der Expansionsfaktor des Katalysatorbetts, d.h. das Verhältnis des vom fluidisierten Katalysatorbett eingenommenen Volumens zum Volumen des Katalysatorbetts ohne Durchströmung.

Bei einem Fliessbett am Lockerungspunkt liegt dieser Faktor bei ≤ 1,15; vorzugsweise < 1,10 und besonders bevorzugt < 1,05. Das Volumen der von Katalysatorpartikeln erfüllten Reaktionszone während der Durchströmung mit Reaktionsmedium ist also um max. 15 %, vorzugsweise max. 10 %, besonders max. 5 % größer ist als im Zustand ohne Durchströmung.

Geeignete Betriebspunkte für das erfindungsgemäße Verfahren unter Ausbildung eines expandierten Fliessbetts liegen bei Leerrohrgeschwindigkeiten deutlich jenseits des Lockerungspunktes. Diese Betriebspunkte führen zu Expansionsfaktoren von 1,01 bis 4; vorzugsweise 1,05 bis 2 und besonders bevorzugt 1,1 bis 1,5 (Verhältnis des vom fluidisierten Katalysatorbett eingenommenen Volumens zum Volumen des Katalysatorbetts ohne Durchströmung). Das Volumen der von Katalysatorpartikein erfüllten Reaktionszone während der Durchströmung mit Reaktionsmedium ist also um 1 bis 300 %, vorzugsweise 5 bis 100 %, besonders vorzugsweise 10 bis 50 % größer als im Zustand ohne Durchströmung.

Das erfindungsgemäße Verfahren wird mit einem zur Ethinylierung nach Reppe geeigneten Kupferacetylid-Katalysator durchgeführt. Diese sind zum Beispiel in DE-A 1 072 985, DE-A 1 075 593, CH-B 220 204, GB-B 784 638, FR-B 1 144 265, DE-B 726 714, DE-B 740 514, DE-B 1 013 279 und GB-B 805 861 beschrieben.

Der Katalysator kann in Pulverform oder vorzugsweise als Formkörper in die Ethinylierungsaktion eingebracht werden. Die Herstellung von Formkörpern aus pulverförmigen Rohstoffen kann durch dem Fachmann bekannte Methoden wie beispielsweise Tablettierung, Agglomeration oder Extrusion erfolgen, wie sie u.a. im Handbook of Heterogenous Catalysis, Vol. 1, VCH Verlagsgesellschaft Weinheim, 1997, S. 414-417 beschrieben sind. Bei der Verformung können dem Fachmann bekannte Hilfsstoffe wie Binder, Schmierstoffe und/oder Lösungsmittel zugesetzt werden. Der Katalysator kann für die Polymerisation z. B. in Form von Zylindern, Strängen, Rippsträngen, Kugeln, Ringen oder Splitt eingesetzt werden. Vorzugsweise werden Kugeln, kugelähnliche Formkörper oder Splitt eingesetzt.

Die Teilchengröße des Katalysators kann je nach Reaktionsbedingungen und Katalysatortyp in weiten Grenzen variiert werden. Üblicherweise haben die einzelnen Katalysatorpartikel für die erfindungsgemäße Fließbettfahrweise eine Größe von 0,2 bis 3 mm, bevorzugt 0,8 bis 1,5 mm.

Die Ethinylierung wird im Allgemeinen bei Temperaturen von 70 bis 120°C, bevorzugt 80 bis 90°C, und einem Betriebsdruck im Reaktionsraum, insbesondere im Ethinylierungsreaktor , von 1 bar bis 15 bar, bevorzugt 3 bis 7 bar, durchgeführt. Als zusammenhängende Gasphase im Sinne der Erfindung werden Gasräume innerhalb des Reaktionsraumes verstanden, die über einzelne, diskrete Blasen oder Bläschen hinausgsehen.

Der wässrigen Formaldehydlösung, in der Acetylen gelöst vorliegt, wird in dem erfindungsgemäßen Verfahren ein mit wässrigem Formaldehyd mischbares organisches Lösungsmittel für Acetylen zugesetzt. Die Konzentration an Formaldehyd in der Lösung beträgt vorteilhaft 1 bis 40 Gew.-%, insbesondere 10 bis 30 Gew.-%, bezogen auf das gesamte Gemisch. Als organisches Lösungsmittel für Acetylen werden solche bevorzugt, die unter Reaktionsbedingungen mehr als 2 cm³ gasförmiges Acetylen je Kubikzentimeter Lösungsmittel aufnehmen. Geeignet sind daher cyclische Äther, wie Tetrahydrofuran, Dimethyltetrahydrofuran, Hexamethylenoxid oder Dioxan, ferner Lactone, wie Butyrolacton, sowie disubstituierte Carbonsäureamide, wie N-Methylpyrrolidon und Dimethylformamid, außerdem Acetale, wie Formaldehyddimethylacetall, und Alkohole, wie Methanol. Besonders bevorzugt wird Tetrahydrofuran als Lösungsmittel verwendet.

Das Gewichtsverhältnis von organischem Lösungsmittel zu Formaldehyd in der wässrigen Formaldehydlösung beträgt dabei von 0,1:1 bis 20:1, vorzugsweise von 1,5:1 bis 4:1.

Die erfindungsgemäße Umsetzung erfolgt in der Flüssigphase mit in einer wässrigen organisches Lösungsmittel enthaltenden Formaldehydlösung gelöstem Acetylen, das in dieser Form dem Reaktionsraum, d.h. dem Ethinylierungsreaktor, zugeführt wird, worin der Partialdruck des Acetylens in der wässrigen, ein organisches Lösungsmittel enthaltenden Formaldehydlösung 0,1 bis 95 % des Betriebsdrucks im Reaktionsraum beträgt.

Die umzusetzende wässriges, organisches Lösungsmittel enthaltende Formaldehydlösung wird vor dem Eintritt in den Reaktionsraum, beispielsweise den Ethinylierungsreaktor, mit Acetylen versetzt, wobei die Acetylenmenge zum Beispiel das 0,1 bis 1fache der jeweiligen Sättigungskonzentration betragen kann. Als umzusetzende Lösung ist in diesem Fall auch die rückgeführte Flüssigkeit bei kontinuierlicher Fahrweise zu verstehen, die noch Formaldehyd enthält, aber an Acetylen verarmt ist.

Die Mischung von Acetylen mit der wässrigen, organisches Lösungsmittel enthaltenden Formaldehydlösung erfolgt in einem geeigneten katalysatorfreien Vorraum des Reaktionsgefässes oder bevorzugt in einer geeigneten Mischdüsenanordnung oder bevorzugt in einem separaten Gas-/Flüssigkeitskontaktapparat, wie zum Beispiel einem als Sättiger dienenden gegebenenfalls mit Packungen, wie zum Beispiel Füllkörpern, gefüllten Reaktionsrohr, durchgeführt werden.

Der pH-Wert der mit Acetylen gesättigten, wässrigen organisches Lösungsmittel enthaltenden Formaldehydlösung wird durch Zudosieren einer 1 bis 5 gew.-%igen Natriumbicarbonatlösung auf 3 bis 8, bevorzugt 6 bis 7, eingestellt. Die pH-Wert-Einstellung kann aber auch beispielsweise mit Soda-Lösung oder Natronlauge erfolgen.

Vorteilhaft kann das Verfahren in einer Apparatur, die mit der nachstehend erläuterten Abbildung beschrieben wird, durchgeführt werden.

Die wässrige, Tetrahydrofuran enthaltende Formaldehydlösung (A) wird über die Förderpumpe (1) in den oberen Teil des mit einer Packung, beispielsweise Füllkörpern, versehenen als Sättiger dienenden Reaktionsrohres (2) geführt. Gegebenenfalls kann die Formaldehydlösung mit Reaktionsaustrag aus der Ethinylierung vermischt sein. Über den nicht gezeigten Verdichter wird Acetylen unter einem Druck von im allgemeinen 4 bis 6 bar von unten in das als Sättiger dienende Reaktionsrohr (2), wobei im Sättiger ein bestimmter Druck von 3 bis 4 bar und eine Temperatur von 70°C bis 85°C aufrechterhalten werden, eingespeist. Über Förderpumpe (3) wird soviel 1 bis 5 gew.-%ige Natriumbicarbonatlösung (B) zudosiert, dass der pH-Wert der den Sättiger (2) über Rohrleitung (4) mittels Pumpe (5) verlassenden Acetylen gesättigten, wässrigen, organisches Lösungsmittel enthaltenden Formaldehydlösung bevorzugt einen pH-Wert von 6 bis 7 aufweist.

Ein Druckregeler (5) regelt dabei den Anlagendruck über den Acetylenzulauf. Eine geringe Menge an Acetylen wird am Kopf des Sättigers mengengeregelt entspannt und geht als Abgas verloren. Dieses Abgas kann prinzipiell, nach entsprechender Anreicherung mit Acetylen und Entfernen des Kohlendioxids, dem Acetylenzulauf wieder zugeführt werden. Das Einsatzgemisch wird aus dem Sumpf des Sättigers (2) mittels Pumpe (5) in den Ethinylierungsreaktor (6) gepumpt. Im Ethinylierungsreaktor (6) wird der Kupferacetylid-Katalysator in Form von 1,5 mm Split (7) durch entsprechende Mengen an umgepumpter Flüssigkeit (Umlaufmenge) im Reaktor in der Schwebe gehalten. Die Umlaufmenge wird dabei so eingestellt, dass sich eine konstante Expansion von bevorzugt x ergibt. Die Reaktion läuft im Wesentlichen isotherm, da die entstehende Reaktionswärme über die Reaktorwand an das im Doppelmantel des Reaktors gefindliche Öl abgegeben wird.

Am Reaktorkopf des Ethinylierungsreaktors erfolgt die über den Sumpfstand des Sättigers (2) geregelte Entspannung des Austrags, der gesammelt und analysiert wird.

Der nach dem Verfahren der Erfindung hergestellte Propargylalkohol ist Ausgangsstoff für die Herstellung von Schädlingsbekämpfungsmitteln und von 2-Aminopyrimidin, das in Arzneimittelsynthesen verwendet wird.

### Beispiele

### Beispiele 1 - 6

Ein zylindrischer Reaktor mit einem Durchmesser von 40 mm und einer Höhe von 2000 mm wird mit 1,5 l Katalysator entsprechend einer Schütthöhe von 1300 mm gefüllt. Der Katalysator hat eine mittlere Partikelgröße von 1,5 mm und enthält 14 Gew.-% Kupfer in Form von Kupferacetylid und 4 Gew.-% Wismut-Oxid, berechnet als Wismut-oxid, auf Siliciumdioxid als Trägermaterial. Der Betriebsdruck beträgt 3,3 bar. Der Reaktor wird mit einer bei einem Acetylenpartialdruck von 3 bar beladenen Formaldehydlösung, die mit Tetrahydrofuran gemäß Tabelle1 versetzt ist, von unten nach oben durchströmt. Der Zulauf des Einsatzgemisches wird dabei so eingestellt, dass der Katalysator in der Schwebe gehalten wird. Der pH-Wert der Zulauflösung beträgt 6 bis 6,5. Die Expansion des Fließbettes beträgt 325mm. Die sonstigen Reaktionsbedingungen sind Tabelle 1, die Zusammensetzung des Austrags die Raum-Zeit-Ausbeuten und das Verhältnis von Propinol zu 1,4-Butindiol im Austrag sind Tabelle 2 zu entnehmen.

### Vergleichsbeispiel 1

Das Vergleichsbeispiel wird wie das erfindungsgemäße Beispiel 1 durchgeführt, jedoch besteht der Zulauf von 313 ml/h aus einer bei einem Acetylenpartialdruck von 3 bar beladenen 30 gew.-%igen Formaldehydlösung, die frei von Tetrahydrofuran ist. Die weiteren Versuchsbedingungen sind Tabelle 1 und das Versuchsergebnis Tabelle 2 zu entnehmen.

**Tabelle 1**

| Bsp. Nr. | Temp. °C | FA [Gew.-%] | Gemisch FA /THF²⁾ [Gew. Gew.-%] | Zulauf FA-Gehalt [Gew.-%] | Zulauf [ml/h] |
|---|---|---|---|---|---|
| 1 | 80 | 30 | 1 / 1 | 15 | 321 |
| 2 | 75 | 30 | 1 / 1 | 15 | 313 |
| 3 | 75 | 30 | 2 / 1 | 20 | 317 |
| 4 | 80 | 30 | 1 / 0 | 30 | 347 |
| 5 | 80 | 49 | 1 / 1 | 25 | 311 |
| 6 | 75 | 49 | 1 / 1 | 25 | 318 |
| V1 | 80 | 30 | 1 / 0 | 30 | 313 |

| | | | | | |
|---|---|---|---|---|---|
| ¹⁾ Formaldehyd ²⁾ Tetrahydrofuran | | | | | |

**Tabelle 2**

| Bsp. Nr. | | **Austrag** | | | **RZA³⁾** | | **Verhältnis** |
|---|---|---|---|---|---|---|---|
| | FA [Gew.-%] | Propinol [Gew.-%] | 1,4-Butindiol [Gew.-%] | Rückstand [Gew.-%] | Propinol [kg/(I_{Kat}.*Tag)] | 1,4-Butindiol [kg/(l_{Kat}.*Tag)] | Propinol/1,4-Butindiol [Gew.-%/Gew.-%] |
| 1 | 3,6 | 1,6 | 13,1 | 0,1 | 0,087 | 0,714 | 10,9 / 89,1 |
| 2 | 5 | 1,66 | 11,8 | 0,1 | 0,088 | 0,624 | 12,4 / 87,6 |
| 3 | 9 | 1,49 | 13,9 | 0,1 | 0,083 | 0,777 | 9,7 / 90,3 |
| 4 | 10,4 | 0,93 | 21,8 | 0,3 | 0,062 | 1,45 | 4,1 / 95,5 |
| 5 | 7,8 | 1,8 | 16,9 | 0,2 | 0,102 | 0,944 | 10,8 / 89,2 |
| 6 | 11,4 | 1,82 | 13,2 | 0,2 | 0,131 | 0,914 | 14,2 / 85,8 |
| V1 | 10,1 | 0,96 | 22,1 | 0,2 | 0,058 | 1,333 | 4,2 / 95,8 |

| | | | | | | | |
|---|---|---|---|---|---|---|---|
| ³⁾ Raum-Zeit-Ausbeute | | | | | | | |

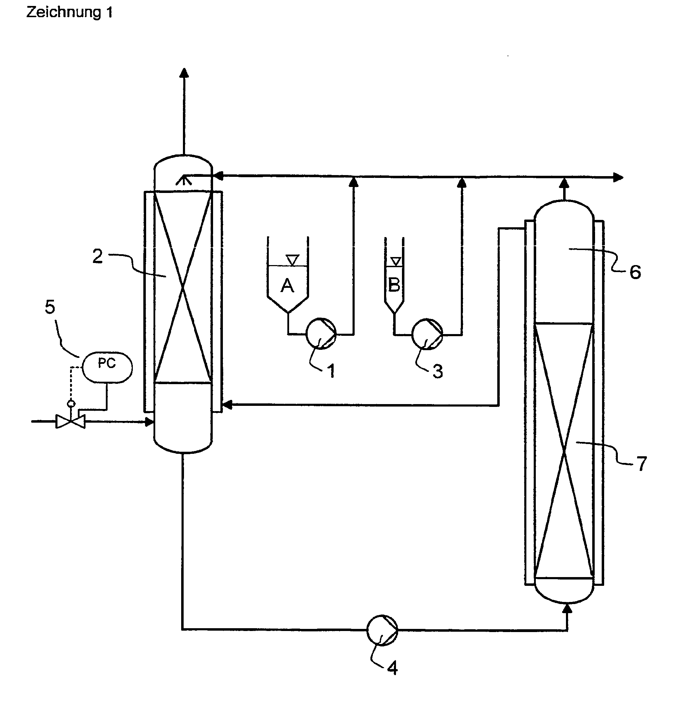

## Patentansprüche

1. Verfahren zur Herstellung von Propargylalkohol durch Umsetzung einer Acetylen enthaltenden wässrigen Formaldehyd-Lösung an einem Kupferacetylid aufweisenden Katalysator bei einem Betriebsdruck von 1 bis 15 bar und 70 bis 120°C ohne Ausbildung einer zusammenhängenden Gasphase, **dadurch gekennzeichnet, dass** die wässrige Formaldehydlösung als organisches Lösungsmittel für Acetylen Tetrahydrofuran enthält und der Katalysator im Fließbett angeordnet ist.

2. Verfahren nach Anspruch 1, **dadurch gekennzeichnet, dass** der Expansionsfaktor des Fließbetts ≤ 1,15 beträgt.

3. Verfahren nach einem der Ansprüche 1 oder 2, **dadurch gekennzeichnet, dass** der Betriebsdruck 3 bis 7 bar beträgt.

4. Verfahren nach einem der Ansprüche 1 bis 3, **dadurch gekennzeichnet, dass** der pH-Wert der wässrigen Formaldehydlösung auf 3 bis 8 eingestellt wird.

5. Verfahren nach einem der Ansprüche 1 bis 4, **dadurch gekennzeichnet, dass** das Gewichtsverhältnis von organischem Lösungsmittel zu Formaldehyd in der wässrigen Formaldehydlösung 0,1 zu 1 bis 20:1 beträgt.

## Claims

1. A process for preparing propargyl alcohol by converting an aqueous formaldehyde solution comprising acetylene over a catalyst comprising copper acetylide at an operating pressure of from 1 to 15 bar and from 70 to 120°C without forming a continuous gas phase, wherein the aqueous formaldehyde solution comprises tetrahydrofuran as the organic solvent for acetylene and the catalyst is arranged in a fluidized bed.

2. The process according to claim 1, wherein the expansion factor of the fluidized bed is ≤1.15.

3. The process according to either of claims 1 and 2, wherein the operating pressure is from 3 to 7 bar.

4. The process according to any of claims 1 to 3, wherein the pH of the aqueous formaldehyde solution is adjusted to from 3 to 8.

5. The process according to any of claims 1 to 4, wherein the weight ratio of organic solvent to formaldehyde in the aqueous formaldehyde solution is from 0.1:1 to 20:1.

## Revendications

1. Procédé de préparation d'alcool propargylique par réaction d'une solution aqueuse de formaldéhyde contenant de l'acétylène sur un catalyseur présentant de l'acétylure de cuivre à une pression de fonctionnement de 1 à 15 bars et à 70 jusqu'à 120°C, sans formation d'une phase gazeuse cohérente, **caractérisé en ce que** la solution aqueuse de formaldéhyde contient, comme solvant organique pour l'acétylène, du tétrahydrofuranne et le catalyseur est agencé dans un lit fluidisé.

2. Procédé suivant la revendication 1, **caractérisé en ce que** le facteur d'expansion du lit fluidisé est ≤ 1,15.

3. Procédé suivant l'une des revendications 1 et 2, **caractérisé en ce que** la pression de fonctionnement est de 3 à 7 bars.

4. Procédé suivant l'une des revendications 1 à 3, **caractérisé en ce que** la valeur de pH de la solution aqueuse de formaldéhyde est ajustée à 3 jusqu'à 8.

5. Procédé suivant l'une des revendications 1 à 4, **caractérisé en ce que** le rapport pondéral entre le solvant organique et le formaldéhyde dans la solution aqueuse de formaldéhyde est de 0,1:1 à 20:1.
